# EUROPEAN PATENT APPLICATION

(11) **EP 4 198 130 A1**
(43) Date of publication of application: **21.06.2023**
(21) Application number: 21867058.6
(22) Date of filing: 06.09.2021
(51) Int. Cl.: C12N 15/70, C07K 14/245, C12N 9/16, C12N 9/04, C12N 9/10, C12P 13/06

(54) **NOVEL O-PHOSPHOSERINE EXPORT PROTEIN AND METHODS FOR PRODUCING O-PHOSPHOSERINE, CYSTEINE, AND CYSTEINE DERIVATIVE USING SAME**

(30) Priority: 09.09.2020 KR 20200115569
(71) Applicant: CJ Cheiljedang Corporation, Seoul 04560 (KR)
(72) Inventor: PARK, Hye Min, Seoul 04560 (KR); KIM, So-Yeon, Seoul 04560 (KR); SIM, Hee-jin, Seoul 04560 (KR); YOO, Hyeryun, Seoul 04560 (KR); LEE, Jin Nam, Seoul 04560 (KR)
(74) Representative: Icosa
(86) International application number: PCT/KR2021/011994
(87) International publication number: WO 2022/055192

(57) **Abstract**

The present disclosure relates to a novel *O*-phosphoserine export protein, and a method for producing *O*-phosphoserine, cysteine, and cysteine derivatives using the same.

## Description

### [Technical Field]

The present disclosure relates to an O-phosphoserine export protein, and a method for producing O-phosphoserine, cysteine, and cysteine derivatives using the same.

### [Background Art]

L-Cysteine, an amino acid which has an important role in sulfur metabolism in all living organisms, is used not only in the synthesis of biological proteins such as hair keratin, *etc.,* glutathione, biotin, methionine, and other sulfur-containing metabolites, but also as a precursor for biosynthesis of coenzyme A.

Methods of producing L-cysteine using microorganisms known in the art include: 1) a method of biologically converting D,L-2-amino-2-thiazoline-4-carboxylic acid (D,L-ATC) into L-cysteine using microorganisms, 2) a method of producing L-cysteine by direct fermentation using *E. coli* (EP 0885962 B; Wada M and Takagi H, Appl. Microbiol. Biochem., 73:48-54, 2006), and 3) a method of producing *O*-phosphoserine (hereinafter, "OPS") by fermentation using microorganisms, and then converting *O*-phosphoserine into L-cysteine by reacting *O*-phosphoserine with a sulfide under the catalytic action of *O*-phosphoserine sulfhydrylase (hereinafter, "OPSS") (European Patent No. 2444481).

In particular, in order to produce cysteine by the method 3) in high yield, OPS, the precursor, should be produced in an excess amount.

Under such circumstances, the present inventors have made extensive efforts to identify a suitable export factor that can smoothly export *O*-phosphoserine produced in an OPS-producing strain outside of cells and to increase the OPS production. As a result, they have discovered a novel OPS export protein, thereby completing the present disclosure.

### [Disclosure]

### [Technical Problem]

The present inventors have made extensive efforts to identify appropriate exporting factors capable of smoothly exporting O-phosphoserine outside of cells and to increase the production of OPS, and as a result, they have found a novel OPS exporting protein, thereby completing the present disclosure.

### [Technical Solution]

It is one object of the present disclosure to provide a recombinant microorganism for producing O-phosphoserine in which the activity of mdtH protein is enhanced compared to its endogenous activity.

It is another object of the present disclosure to provide a method for producing *O*-phosphoserine using the recombinant microorganism for producing *O*-phosphoserine of the present disclosure.

It is still another object of the present disclosure to provide a method for producing cysteine or derivatives thereof using the recombinant microorganism for producing *O*-phosphoserine of the present disclosure.

### [Advantageous Effects]

When *O*-phosphoserine is produced using the recombinant microorganism for producing *O*-phosphoserine in which the activity of mdtH protein is enhanced compared to its endogenous activity, it can lead to high-yield production of *O*-phosphoserine compared to using an existing non-modified strain.

### [Detailed Description of Preferred Embodiments]

The present disclosure will be described in detail. Meanwhile, each description and embodiment disclosed herein can be applied to other descriptions and embodiments, respectively. That is, all combinations of various elements disclosed herein fall within the scope of the present disclosure. Further, the scope of the present disclosure is not limited by the specific description described below.

In one aspect of the present disclosure to achieve the objects above, the present disclosure provides a recombinant microorganism for producing *O*-phosphoserine in which the activity of mdtH protein is enhanced compared to its endogenous activity.

As used herein, the term "*O*-phosphoserine" (hereinafter, "OPS") refers to a phosphoric acid ester of serine which serves as a constituting component for many proteins. In particular, the OPS is a precursor of L-cysteine and can be converted to cysteine by reacting with a sulfide under the catalytic action of OPS sulfhydrylase (hereinafter, "OPSS"), but is not limited thereto (European Patent No. 2444481).

Specifically, the recombinant microorganism of the present disclosure may have an enhanced *O*-phosphoserine exporting activity compared to its endogenous activity. The mdtH protein of the present disclosure may have an activity of exporting O-phosphoserine outside of cells, and such activity has been identified in the present disclosure for the first time.

In one example, the mdtH protein of the present disclosure may be a membrane protein, and may be derived from *Escherichia coli.*

Additionally, the mdtH protein of the present disclosure may be a transporter belonging to the major facilitator superfamily (MFS).

Specifically, the mdtH protein of the present disclosure may be a protein including an amino acid sequence of SEQ ID NO: 1, or an amino acid sequence having at least 95% identity with SEQ ID NO: 1, while having the *O*-phosphoserine exporting activity. For example, the protein including the amino acid having at least 95% identity with the amino acid sequence of SEQ ID NO: 1 may include any protein which expresses an OPS exporting activity identical or corresponding to the mdtH protein including the amino acid sequence of SEQ ID NO: 1 without limitation.

More specifically, the amino acid having at least 95% identity with the amino acid sequence of SEQ ID NO: 1 may be a sequence having an identity of 96% or more, 97% or more, 98% or more, 99% or more, 99.5% or more, or 99.75% or more, and 100% or less to the amino acid sequence of SEQ ID NO: 1.

Additionally, the mdtH protein of the present disclosure may include protein variants, in which a part of the sequence is deleted, modified, substituted, conservatively substituted, or added in the amino acid sequence of SEQ ID NO: 1 or the amino acid sequence having at least 95% identity with the amino acid sequence of SEQ ID NO: 1, as long as they have an *O*-phosphoserine exporting activity, and may substantially fall within the scope of the present disclosure.

Additionally, it is apparent to those skilled in the art that the mdtH protein of the present disclosure may include a meaningless sequence addition upstream or downstream of the amino acid sequence of SEQ ID NO: 1 or the amino acid sequence having at least 95% identity with the amino acid sequence of SEQ ID NO: 1 while having the *O*-phosphoserine exporting activity, a naturally occurring mutation, or a silent mutation thereof.

As used herein, the term "homology" or "identity" refers to a degree of relatedness between two given amino acid sequences or nucleotide sequences, and may be expressed as a percentage. The terms homology and identity may often be used interchangeably with each other.

The sequence homology or identity of conserved polynucleotide or polypeptide may be determined by standard alignment algorithms and can be used with a default gap penalty established by the program being used. Substantially, homologous or identical sequences are generally expected to hybridize to all or part of the sequences under moderate or high stringent conditions. It is apparent that hybridization with polynucleotides containing general codon or degenerate codons in hybridizing polynucleotides is also included.

Whether any two polynucleotide or polypeptide sequences have a homology, similarity, or identity may be, for example, determined by a known computer algorithm such as the "FASTA" program (Pearson et al., (1988) Proc. Natl. Acad. Sci. USA 85:2444) using default parameters. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (preferably, version 5.0.0 or later) (GCG program package (Devereux, J. et al., Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, the homology, similarity, or identity may be determined using BLAST or ClustalW of the National Center for Biotechnology Information (NCBI).

The homology, similarity, or identity of polynucleotides or polypeptides may be, for example, determined by comparing sequence information using, for example, the GAP computer program, such as Needleman et al. (1970), J Mol Biol. 48:443 as disclosed in Smith and Waterman, Adv. Appl. Math (1981) 2:482. In summary, the GAP program defines the homology, similarity, or identity as the value obtained by dividing the number of similarly aligned symbols (*i.e*., nucleotides or amino acids) by the total number of the symbols in the shorter of the two sequences. Default parameters for the GAP program may include (1) a unary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

As used herein, the term "a recombinant microorganism for producing O-phosphoserine (OPS)" may refer to a microorganism that has a naturally weak OPS producing ability or a microorganism that has been provided with an OPS producing ability by way of natural or artificial genetic modification of a parent strain that does not have an OPS producing ability. In the present disclosure, the "recombinant microorganism for producing OPS" may be used interchangeably with "a microorganism having an OPS producing ability".

For the purpose of the present disclosure, in the case of the OPS-producing microorganism, the production amount of OPS may be increased compared to that of a wild-type or a microorganism before modification, as the activity of mdtH protein is enhanced. This is significant in that the OPS producing ability may be increased by enhancing the activity of mdtH protein of the present disclosure, while wild-type microorganisms or microorganisms before modification cannot produce OPS or can only produce trace amounts even if they are able to produce OPS.

As used herein, the term "enhancement" of a polypeptide activity means that the activity of a polypeptide is increased compared to its endogenous activity. The enhancement may be used interchangeably with terms such as activation, up-regulation, overexpression, increase, *etc.* In particular, the activation, enhancement, up-regulation, overexpression and increase may include both cases in which an activity not originally possessed is exhibited, or the activity is enhanced compared to the endogenous activity or the activity before modification. The "endogenous activity" refers to the activity of a particular polypeptide originally possessed by a parent strain before transformation or a non-modified microorganism, when a trait is altered through genetic modification caused by natural or artificial factors, and may be used interchangeably with "activity before modification". The "enhancement", "up-regulation", "overexpression" or "increase" in the activity of a polypeptide compared to its endogenous activity means that the activity and/or concentration (expression level) of the polypeptide is enhanced compared to that of a particular polypeptide originally possessed by a parent strain before transformation or a non-modified microorganism.

The enhancement may be achieved by introducing a foreign polypeptide, or by enhancing the activity and/or concentration (expression level) of the endogenous polypeptide. The enhancement of the activity of the polypeptide can be confirmed by the increase in the level of activity of the polypeptide, expression level, or the amount of product excreted from the polypeptide.

The enhancement of the activity of the polypeptide can be applied by various methods well known in the art, and is not limited as long as it can enhance the activity of the target polypeptide compared to that of the microorganism before modification. Specifically, genetic engineering and/or protein engineering well known to those skilled in the art, which is a common method of molecular biology, may be used, but the method is not limited thereto (*e.g*., Sitnicka et al. Functional Analysis of Genes. Advances in Cell Biology. 2010, Vol. 2. 1-16; Sambrook et al. Molecular Cloning 2012, *etc*.).

Specifically, the enhancement of the polypeptide of the present application may be achieved by:
1) increasing the intracellular copy number of a polynucleotide encoding the polypeptide;
2) modifying the expression regulatory region of a gene encoding the polypeptide on the chromosome (*e.g*., inducing a modification on the expression regulatory region, replacing the sequence with a sequence having a stronger activity, or insertion of a sequence having a stronger activity);
3) modifying a nucleotide sequence encoding the initiation codon or 5'-UTR of the gene transcript encoding the polypeptide;
4) modifying the amino acid sequence of the polypeptide such that the activity of the polypeptide is enhanced;
5) modifying the polynucleotide sequence encoding the polypeptide such that the activity of the polypeptide is enhanced (*e.g*., modifying the polynucleotide sequence of the polypeptide gene to encode a polypeptide that has been modified to enhance the activity of the polypeptide);
6) introducing a foreign polypeptide exhibiting the polypeptide activity or a foreign polynucleotide encoding the same;
7) codon-optimization of the polynucleotide encoding the polypeptide;
8) analyzing the tertiary structure of the polypeptide and thereby selecting and modifying the exposed site, or chemically modifying the same; or
9) a combination of two or more selected from above 1 to 8), but is not particularly limited thereto.

More specifically:
The 1) method of increasing the intracellular copy number of a polynucleotide encoding the polypeptide may be achieved by introducing a vector, which is operably linked to the polynucleotide encoding the polypeptide and is able to replicate and function regardless of a host cell, into the host cell. Alternatively, the method may be achieved by introducing one copy or two copies of polynucleotides encoding the polypeptide into the chromosome of a host cell. The introduction into the chromosome may be performed by introducing a vector, which is able to insert the polynucleotide into the chromosome of a host cell, into the host cell, but is not limited thereto. The vector is as described above.
The 2) method of replacing the expression regulatory region (or expression regulatory sequence) of a gene encoding the polypeptide on the chromosome with a sequence having a strong activity may be achieved, for example, by inducing a modification on the sequence through deletion, insertion, non-conservative or conservative substitution, or a combination thereof to further enhance the activity of the expression regulatory region, or by replacing the sequence with a sequence having a stronger activity. The expression regulatory region may include, but is not particularly limited to, a promoter, an operator sequence, a sequence encoding a ribosome binding site, and a sequence regulating the termination of transcription and translation, *etc.* In one example, the method may include replacing the original promoter with a strong promoter, but is not limited thereto.

Examples of the known strong promoter may include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc.,* but the strong promoter is not limited thereto.

The 3) method of modifying a nucleotide sequence encoding the initiation codon or 5'-UTR of the gene transcript encoding the polypeptide may be achieved, for example, by substituting the nucleotide sequence with a nucleotide sequence encoding another initiation codon having a higher expression rate of the polypeptide compared to the endogenous initiation codon, but is not limited thereto.

The 4) and 5) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to enhance the activity of the polypeptide, or by replacing the sequence with an amino acid sequence or polynucleotide sequence modified to have a stronger activity, or an amino acid sequence or polynucleotide sequence modified to enhance the activity, but are not limited thereto. The replacement may specifically be performed by inserting the polynucleotide into the chromosome by homologous recombination, but is not limited thereto. The vector used herein may further include a selection marker to confirm the insertion into the chromosome. The selection marker is as described above.

The 6) method of introducing a foreign polynucleotide exhibiting the activity of the polypeptide may be achieved by introducing into a host cell a foreign polynucleotide encoding a polypeptide that exhibits the same/similar activity to that of the polypeptide. The foreign polynucleotide may be used without limitation regardless of its origin or sequence as long as it exhibits the same/similar activity to that of the polypeptide. The introduction may be performed by those of ordinary skill in the art by appropriately selecting a transformation method known in the art, and the expression of the introduced polynucleotide in the host cell enables to produce the polypeptide, thereby increasing its activity.

The 7) method of codon-optimization of the polynucleotide encoding the polypeptide may be achieved by codon-optimization of an endogenous polynucleotide to increase the transcription or translation within a host cell, or by optimizing the codons thereof such that the optimized transcription and translation of the foreign polynucleotide can be achieved within the host cell.

The 8) method of analyzing the tertiary structure of the polypeptide and thereby selecting and modifying the exposed site, or chemically modifying the same may be achieved, for example, by comparing the sequence information of the polypeptide to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

Such enhancement of the polypeptide activity may mean that the activity or concentration of the corresponding polypeptide is increased relative to the activity or concentration of the polypeptide expressed in a wild-type or a microorganism before modification, or that the amount of product produced from the polypeptide is increased, but is not limited thereto.

As used herein, the term "vector" may include a DNA construct containing a nucleotide sequence of a polynucleotide encoding the target polypeptide operably linked to an expression regulatory region (or expression regulatory sequence) suitable for expressing the target polypeptide in a suitable host. The expression regulatory region may include a promoter capable of initiating transcription, any operator sequence for regulating the transcription, a sequence encoding a suitable mRNA ribosome binding site, and a sequence for regulating termination of transcription and translation. Once transformed into a suitable host cell, the vector may replicate or function independently from a host genome, or may integrate into the genome.

The vector used in the present disclosure is not particularly limited, and any vector known in the art may be used. Examples of conventional vectors may include a natural or recombinant plasmid, cosmid, virus and bacteriophage. For example, pWE15, M13, MBL3, MBL4, IXII, ASHII, APII, t10, t11, Charon4A, and Charon21A may be used as a phage vector or a cosmid vector. As a plasmid vector, pDZ type, pBR type, pUC type, pBluescriptII type, pGEM type, pTZ type, pCL type, and pET type may be used. Specifically, pDZ, pDC, pDCM2, pACYC177, pACYC184, pCL, pECCG117, pUC19, pBR322, pMW118, and pCC1 BAC vectors may be used.

For example, the polynucleotide encoding the target polypeptide may be inserted into a chromosome using a vector for chromosomal insertion. The insertion of the polynucleotide into the chromosome may be performed by way of any method known in the art, for example, homologous recombination, without being limited thereto. The polynucleotide may further include a selection marker to confirm the chromosomal insertion. The selection marker is used to select cells that are transformed with the vector, that is, to confirm insertion of a desired nucleic acid molecule, and examples of the selection marker may include markers providing selectable phenotypes, such as drug resistance, nutrient requirement, resistance to cytotoxic agents, or expression of surface polypeptide. Only cells expressing the selection marker are able to survive or to show different phenotypes under the environment treated with a selective agent, and thus the transformed cells may be selected.

As used herein, the term "transformation" refers to a process of introducing a vector including a polynucleotide encoding a target polypeptide into a host cell or microorganism in such a way that the polypeptide encoded by the polynucleotide is expressed in the host cell. The transformed polynucleotide may be either in a form inserted into the chromosome of the host cell or in a form located outside the chromosome as long as the protein is expressed in the host cell. In addition, the polynucleotide includes DNA and/or RNA encoding the target polypeptide. The polynucleotide may be introduced into the host cell in any form as long as the polynucleotide is introduced into the host cell and the polypeptide is expressed therein. For example, the polynucleotide may be introduced into the host cell in the form of an expression cassette that is a gene construct including all of the essential elements required for self-replication. The expression cassette may generally include a promoter operably linked to the polynucleotide, a transcription termination signal, a ribosome binding site, and a translation termination signal. The expression cassette may be in the form of a self-replicable expression vector. Also, the polynucleotide may be introduced into the host cell in its original form and operably linked to a sequence required for the expression in the host cell, without being limited thereto.

In addition, as used herein, the term "operably linked" refers to an operable linkage between a promoter sequence, which enables initiation and mediation of transcription of a polynucleotide encoding the target variant of the present disclosure, and the polynucleotide sequence.

Specifically, the recombinant microorganism of the present disclosure may be a microorganism introduced with a gene/polynucleotide encoding the mdtH protein; a microorganism in which the intracellular copy number of the polynucleotide encoding the mdtH protein is increased; a microorganism transformed with a vector containing the polynucleotide encoding the mdtH protein; a microorganism in which the expression regulatory sequence of the gene on the chromosome encoding the mdtH protein is replaced with a sequence with stronger activity; or a microorganism in which the promoter on the chromosome encoding the mdtH protein is replaced with a promoter with strong activity.

More specifically, the gene encoding the mdtH protein may be an mdtH gene. Additionally, the polynucleotide encoding the mdtH protein may be a polynucleotide including the nucleotide sequence of SEQ ID NO: 2. The sequences of SEQ ID NO: 1 and SEQ ID NO: 2 can be obtained from GenBank of the NCBI, a known database.

The protein/polypeptide including an amino acid sequence of a specific sequence number or an amino acid sequence described by a specific sequence number may be a protein/polypeptide having the amino acid sequence of the specific sequence number or the amino acid sequence described by the specific sequence number, or may be a protein/polypeptide consisting or consisting essentially of the amino acid sequence of the specific sequence number or the amino acid sequence described by the specific sequence number. Additionally, the polynucleotide including the nucleotide sequence of the specific sequence number or the nucleotide sequence described by the specific sequence number may be a polynucleotide having the nucleotide sequence of the specific sequence number or the nucleotide sequence described by the specific sequence number, or may be a polynucleotide consisting or consisting essentially of the nucleotide sequence of the specific sequence number or the nucleotide sequence described by the specific sequence number.

The microorganism of the present disclosure is not limited by its type as long as it can produce OPS, and may be any prokaryotic or eukaryotic microorganism, specifically a prokaryotic microorganism. Examples of the prokaryotic microorganism may include microbial strains belonging to the genus *Escherichia,* the genus *Erwinia,* the genus *Serratia,* the genus *Providencia,* the genus *Corynebacterium,* and the genus *Brevibacterium,* specifically a microorganism belonging to the genus *Escherichia,* and more specifically *Escherichia coli,* but is not limited thereto. In particular, in the case of the microorganism belonging to the genus *Escherichia* or *Corynebacterium,* OPS and L-serine can be produced (Ahmed Zahoor, Computational and structural biotechnology journal, Vol. 3, 2012 October; Wendisch, V. F. et al., Curr Opin Microbiol. 2006 Jun;9(3):268-74; Peters-Wendisch, P. et al., Appl Environ Microbiol. 2005 Nov;7 1(II):7 139-44.).

The recombinant microorganism of the present disclosure may be one in which the activity of phosphoserine phosphatase (SerB) is further weakened compared to its endogenous activity.

The SerB of the present disclosure has an activity of converting OPS to L-serine, and thus, the microorganism modified such that the SerB activity is weakened has the property of accumulating OPS therein, and is thereby useful for the production of OPS. The SerB of the present disclosure may be a protein including an amino acid sequence described by SEQ ID NO: 3, but is not limited thereto. Additionally, the SerB may include an amino acid sequence having an identity of 80% or higher, specifically 90% or higher, more specifically 95% or higher, or even more specifically 99% or higher to the amino acid sequence described by SEQ ID NO: 3, as long as it shows the SerB activity, but is not limited thereto. In addition, the polynucleotide encoding the SerB may have a nucleotide sequence encoding the amino acid described by SEQ ID NO: 3. Various modifications may be made in a coding region of the polynucleotide within a range not changing the amino acid sequence of the protein due to codon degeneracy or in consideration of a codon preferred by an organism in which the protein is to be expressed. The polynucleotide encoding SerB of the present disclosure may, for example, include a nucleotide sequence of SEQ ID NO: 4, or a nucleotide sequence having an identity of 80% or higher, specifically 90% or higher, more specifically 95% or higher, or even more specifically 99% or higher to the nucleotide sequence of SEQ ID NO: 4, but is not limited thereto.

As used herein, the term "weakening" of a polypeptide has a concept including all of the decrease or absence of activity compared with the inherent activity. The weakening may be used interchangeably with inactivation, deficiency, down-regulation, decrease, reduction, attenuation, or the like.

The weakening may also include: a case where the activity of the polypeptide itself is decreased or eliminated compared with the activity of the polypeptide itself possessed by the original microorganism due to the mutation or the like of the polynucleotide encoding the polypeptide; a case where the activity and/or concentration (expression level) of the entire polypeptides in the cell is lower compared with the native strain due to the inhibition of the expression of a gene of a polynucleotide encoding the polypeptide or the inhibition of the translation into the polypeptide; a case where the expression of the polynucleotide is not made; and/or a case where the polypeptide has no activity in spite of the expression of the polynucleotide. The "endogenous activity" refers to the activity of a specific polypeptide originally possessed by a parent strain before modification or a wild-type or non-modified microorganism when transformation occurs due to genetic variation caused by native or artificial factors. This term may be used interchangeably with the "activity before modification". The "weakening", "inactivation", "deficiency", "decrease", "down-regulation", "reduction", or "attenuation" of the activity of a polypeptide compared with the endogenous activity means that the activity of the polypeptide is lowered compared with the activity of a specific polypeptide originally possessed by a parent strain before modification or a non-modified microorganism.

The weakening of the activity of the polypeptide may be attained by any method known in the art, but is not limited thereto, and may be attained by applying various methods well known in the art (*e.g*., Nakashima N et al., Bacterial cellular engineering by genome editing and gene silencing. Int J Mol Sci. 2014;15(2):2773-2793, Sambrook et al., Molecular Cloning 2012, *etc*.).

Specifically, the weakening of the polypeptide of the present disclosure may be:
1) a deletion of a part or the entirety of the gene encoding the polypeptide;
2) a modification of an expression control region (or expression control sequence) so as to reduce the expression of the gene encoding the polypeptide;
3) a modification of an amino acid sequence constituting the polypeptide so as to eliminate or weaken the activity of the polypeptide (*e.g*., deletion/substitution/addition of at least one amino acid on the amino acid sequence).
4) a modification of the gene sequence encoding the polynucleotide so as to eliminate or weaken the activity of the polypeptide (*e.g*., deletion/substitution/addition of at least one nucleotide on the nucleotide sequence of the polypeptide gene so as to encode the polypeptide modified to eliminate or weaken the activity of the polypeptide);
5) a modification of a nucleotide sequence encoding the initiation codon or 5'-UTR region of the gene transcript encoding the polypeptide;
6) an introduction of an antisense oligonucleotide (*e.g*., antisense RNA) complementarily binding to the gene transcript encoding the polypeptide;
7) an addition of a sequence complementary to the Shine-Dalgarno sequence of the gene encoding the polypeptide upstream of the Shine-Dalgarno sequence so as to form a secondary structure that makes the attachment of ribosomes impossible;
8) an addition of a reverse transcription promoter to the 3' end of the open reading frame (ORF) of the gene sequence encoding the polypeptide (reverse transcription engineering, RTE); or
9) a combination of two or more selected from items 1) to 8), but is not particularly limited thereto.

For example, these are described as follows.

The deletion of a part or the entirety of the gene encoding the polypeptide in item 1) may be an elimination of the entirety of the polynucleotide encoding an endogenous target protein in the chromosome, a replacement with a polynucleotide with a deletion of some nucleotides, or a replacement with a marker gene.

The modification of an expression control region (or expression control sequence) in item 2) may be a mutation on the expression control region (or expression control sequence) through deletion, insertion, non-conservative or conservative substitution, or a combination thereof, or a replacement with a sequence having weaker activity. The expression control region includes a promoter, an operator sequence, a sequence for encoding a ribosomal binding site, and a sequence for controlling the termination of transcription and translation, but is not limited thereto.

The modification of the nucleotide sequence encoding the initiation codon or 5'-UTR region of the gene transcript encoding the polypeptide in item 3) may be, for example, a substitution with a nucleotide sequence encoding, rather than the endogenous initiation codon, another initiation codon having a lower expression rate of the polypeptide, but is not limited thereto.

The modification of the amino acid sequence or the polynucleotide sequence in items 4) and 5) may be a modification on the sequence through deletion, insertion, non-conservative or conservative substitution, or a combination thereof in the amino acid sequence of the polypeptide or the polynucleotide sequence encoding the polypeptide, so as to weaken the activity of the polypeptide, or a replacement with an amino acid sequence or polynucleotide sequence modified to have weaker activity or an amino acid sequence or polynucleotide sequence modified to have no activity, but is not limited thereto. For example, the expression of the gene may be inhibited or attenuated by introducing a mutation into the polynucleotide sequence to form a termination codon.

The introduction of an antisense oligonucleotide (*e.g*., antisense RNA) complementarily binding to the gene transcript encoding the polypeptide in item 6) may be referred to, for example, the literature (Weintraub, H. et al., Antisense-RNA as a Genetics, Vol. 1(1) 1986).

The addition of a sequence complementary to the Shine-Dalgarno sequence of the gene encoding the polypeptide upstream of the Shine-Dalgarno sequence so as to form a secondary structure that makes the attachment of ribosomes impossible in item 7) may make mRNA translation impossible or reduce the rate thereof.

The addition of a reverse transcription promoter to the 3' end of the open reading frame (ORF) of the gene sequence encoding the polypeptide (reverse transcription engineering, RTE) in item 8) may make an antisense nucleotide complementary to the transcript of the gene encoding the polypeptide to thereby weaken the activity of the polypeptide.

The modification of a part or the entirety of the polynucleotide in the microorganism of the present disclosure may be induced by: (a) genome editing adopting homologous recombination or engineered nuclease (*e.g*., CRISPR-Cas9) using a vector for chromosomal insertion in a microorganism or and/or (b) the treatment with light, such as ultraviolet light and radiation, and/or chemicals, but is not limited thereto. The method of modifying a part or the entirety of the gene may include a method using DNA recombinant technology. For example, a nucleotide sequence or vector containing a nucleotide sequence homologous to a target gene may be introduced into the microorganism to bring about homologous recombination, resulting in a deletion in a part or the entirety of the gene. The nucleotide sequence or vector to be introduced may include a dominant selection marker, but is not limited thereto.

Additionally, the recombinant microorganism of the present disclosure may be one in which the activity of any one of phosphoglycerate dehydrogenase (SerA), phosphoserine aminotransferase (SerC) or a combination thereof is further enhanced compared to its endogenous activity.

The SerA of the present disclosure is a protein having the activity of converting 3-phosphoglycerate into 3-phospho-hydroxypyruvate. The SerC of the present disclosure is a protein having the activity of converting 3-phospho-hydroxypyruvate into OPS. Accordingly, any microorganism with enhanced SerA and/or SerC activity may be effectively used as an OPS-producing microorganism.

The SerA of the present disclosure may be a protein including an amino acid sequence of SEQ ID NO: 5 or 6. The amino acid sequence of SEQ ID NO: 5 is a sequence of the wild-type SerA, and the amino acid sequence of SEQ ID NO: 6 is a sequence of a SerA variant where the feedback inhibition on serine is released. Additionally, the SerA may include an amino acid sequence having an identity of 80% or higher, specifically 90% or higher, more specifically 95% or higher, or even more specifically 99% or higher to the amino acid sequence described by SEQ ID NO: 5 or 6, as long as it shows the activity of the wild-type SerA or the activity of the SerA variant in which the feedback inhibition on serine is released, but is not limited thereto. The SerA variants in which the feedback inhibition on serine is released refer to those proteins in which a modification is introduced on the SerA-encoding gene by insertion, substitution, *etc.,* thereby maintaining the activity from the feedback inhibition by serine or glycine, or having enhanced activities thereof, and those SerA variants where the feedback inhibition on serine is released are already well known (Grant, G. A. et al., J. Biol. Chem., 39:5357-5361, 1999; Grant, G. A. et al., Biochem., 39:7316-7319, 2000; Grant, G. A. et al., J. Biol. Chem., 276:17844-17850, 2001; Peters-Wendisch, P. et al., Appl. Microbiol. Biotechnol, 60:37-441, 2002; EP 0943687 B).

Additionally, the polynucleotide encoding the wild-type SerA or the SerA variant where the feedback inhibition on serine is released may include a nucleotide sequence encoding any one amino acid sequence described by SEQ ID NO: 5 or 6, but is not limited thereto. The polynucleotide sequence encoding the wild-type SerA or the SerA variant where the feedback inhibition on serine is released may undergo various modifications in the coding region within the scope that does not change the amino acid sequence of the polypeptide, due to codon degeneracy or in consideration of the codons preferred in an organism in which the polypeptide is to be expressed. The polynucleotide encoding the wild-type SerA or the SerA variant where the feedback inhibition on serine is released may be, for example, a polynucleotide including a nucleotide sequence of SEQ ID NO: 7 or 8, or may be a polynucleotide including a nucleotide sequence having a homology of 80% or higher, specifically 90% or higher, more specifically 95% or higher, or even more specifically 99% or higher to the nucleotide sequence of SEQ ID NO: 7 or 8, but is not limited thereto.

The SerC may be, for example, a protein including an amino acid sequence described by SEQ ID NO: 9, but is not limited thereto. Additionally, the SerC may include an amino acid sequence having an identity of 80% or higher, specifically 90% or higher, more specifically 95% or higher, or even more specifically 99% or higher to the amino acid sequence described by SEQ ID NO: 9, as long as it shows the activity of SerC, but is not limited thereto.

In addition, the polynucleotide encoding the SerC may include a nucleotide sequence encoding the amino acid sequence described by SEQ ID NO: 9. The polynucleotide encoding the SerC may undergo various modifications in the coding region within the scope that does not change the amino acid sequence of the polypeptide, due to codon degeneracy or in consideration of the codons preferred in an organism in which the polypeptide is to be expressed. The polynucleotide encoding the SerC may include, for example, a nucleotide sequence of SEQ ID NO: 10, or a nucleotide sequence having a homology of 80% or higher, specifically 90% or higher, more specifically 95% or higher, or even more specifically 99% or higher to the amino acid sequence of SEQ ID NO: 10, but is not limited thereto.

As used herein, the term "enhancement compared to its endogenous activity" and the enhancement method are the same as described above.

Additionally, the recombinant microorganism of the present disclosure may be a microorganism in which its capability to introduce OPS into a cell or decompose OPS is further weakened.

Regarding the contents of the OPS-producing microorganism, the disclosures in European Patent No. 2444481 or U.S. Patent Application Publication No. 2012-0190081 may be used as references of the present disclosure, in addition to those described above.

In another aspect of the present disclosure, the present disclosure provides a method for producing *O*-phosphoserine, including culturing a recombinant microorganism for producing *O*-phosphoserine in which the activity of mdtH protein is enhanced compared to its endogenous activity, in a medium.

The mdtH protein, endogenous activity, enhancement, *O*-phosphoserine, and microorganism are as described above.

As used herein, the term "cultivation" means that the microorganism is grown under appropriately controlled environmental conditions. The cultivation process of the present disclosure may be performed in a suitable culture medium and culture conditions known in the art. Such a cultivation process may be easily adjusted for use by those skilled in the art according to the strain to be selected. Specifically, the cultivation may be a batch culture, a continuous culture, and a fed-batch culture, but is not limited thereto.

In the cultivation of the recombinant microorganism in which the SerB activity is weakened compared to its endogenous activity, the medium may further contain glycine or serine, as the serine requirement of the microorganism is induced. Glycine may be provided in the form of purified glycine, a glycine-containing yeast extract, or tryptone. The concentration of glycine to be contained in the medium is generally 0.1 g/L to 10 g/L, and specifically 0.5 g/L to 3 g/L. Additionally, serine may be provided in the form of purified serine, a serine-containing yeast extract, or tryptone. The concentration of serine to be contained in the medium is generally 0.1 g/L to 5 g/L, and specifically 0.1 g/L to 1 g/L.

Examples of the carbon source to be contained in the medium may include saccharides and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These carbon sources may be used alone or in combination, but are not limited thereto.

Examples of the nitrogen source to be contained in the medium may include organic nitrogen sources such as peptone, yeast extract, meat gravy, malt extract, corn steep liquor, and bean flour; and inorganic nitrogen sources such as urea, ammonium sulfate, ammonium chloride, ammonium phosphate, ammonium carbonate, and ammonium nitrate. These nitrogen sources may be used alone or in combination, but are not limited thereto.

Examples of the phosphorous source to be contained in the medium may include potassium dihydrogen phosphate, dipotassium hydrogen phosphate, and corresponding sodium-containing salts, but are not limited thereto.

Additionally, the culture medium may include metal salts, such as magnesium sulfate or iron sulfate, and may further contain amino acids, vitamins, and appropriate precursors. These culture media or precursors may be added to the culture in the form of a batch culture or continuous culture, but are not limited thereto.

The pH of the culture may be adjusted by adding a compound such as ammonium hydroxide, potassium hydroxide, ammonia, phosphoric acid, and sulfuric acid during cultivation in an appropriate manner. Additionally, bubble formation may be prevented during the cultivation using an antifoaming agent such as a fatty acid polyglycol ester. Further, oxygen gas or a gas containing oxygen may be injected to the culture in order to maintain aerobic conditions of the culture; or anaerobic or microaerobic conditions may be maintained without the injection of gas or by injecting nitrogen, hydrogen, or carbon dioxide. The temperature of the culture may be in the range from 25°C to 40°C, and specifically from 30°C to 35°C. The cultivation may be continued until the production of a desired material can be obtained, and may be specifically carried out for from 10 hours to 100 hours, but is not limited to these illustrative examples.

The method of producing O-phosphoserine according to the present disclosure may further include preparing the microorganism of the present disclosure, preparing a culture medium for culturing the strain, or any combination thereof (regardless of order, in any order), for example, before the culturing step.

The method of producing O-phosphoserine according to the present disclosure may further include recovering O-phosphoserine from the culture medium after culturing (culture medium where the culturing is performed) or the microorganism of the present disclosure. The recovering step may further be included after the culturing step.

The recovering may be collecting desired O-phosphoserine using the culturing method of the present disclosure, e.g., an appropriate method known in the art such as a batch, continuous, or fed-batch method. For example, centrifugation, filtration, treatment with a protein precipitating agent (salting out), extraction, ultrasonic disintegration, ultrafiltration, dialysis, various chromatographic methods such as molecular sieve chromatography (gel permeation), adsorption chromatography, ion-exchange chromatography, and affinity chromatography, high-performance liquid chromatography (HPLC), any combination thereof may be used, and the desired O-phosphoserine may be recovered from the culture medium or the microorganism using an appropriate method well known in the art.

In addition, the method of producing O-phosphoserine of the present disclosure may further include purifying the O-phosphoserine. The purifying step may be performed using an appropriate method well known in the art. In an embodiment, when the method of producing O-phosphoserine of the present disclosure includes both the recovering and purifying steps, the recovering and purifying steps may be performed continuously or discontinuously regardless of order, or may be performed simultaneously or as one integrated step, without being limited thereto.

In the method of the present disclosure, the variant, the polynucleotide, the vector, the microorganism, and the like are as described above.

In still another aspect of the present disclosure, the present disclosure provides a method for producing cysteine or derivatives thereof, including:
a) producing *O*-phosphoserine or a medium containing *O*-phosphoserine by culturing a recombinant microorganism for producing *O*-phosphoserine in which the activity of mdtH protein is enhanced compared to its endogenous activity, in a medium; and
b) reacting the *O*-phosphoserine or the medium containing the same produced in Step a) with a sulfide, in the presence of *O*-phosphoserine sulfhydrylase (OPSS) or a microorganism containing the same.

As used herein, "include/including" a specific protein with respect to the microorganism means a state in which a specific protein of interest is introduced into the microorganism or expressed in the microorganism.

The mdtH protein, endogenous activity, enhancement, *O*-phosphoserine, and microorganism are as described above.

As used herein, the term "derivative" refers to similar compounds obtained by chemically modifying a portion of any compound. The term usually refers to compounds in which a hydrogen atom or a particular atom group is substituted with another atom or atom group.

As used herein, the term "cysteine derivative" refers to compounds in which a hydrogen atom or a particular atom group of cysteine is substituted with another atom or atom group. For example, the cysteine derivatives may have a form in which the nitrogen atom of the amine group (-NH₂) or the sulfur atom of the thiol group (-SH) in cysteine has another atom or atom group attached thereto, and examples of cysteine derivatives may include NAC (*N*-acetylcysteine), SCMC (*S*-carboxymethylcysteine), Boc-Cys(Me)-OH, (*R*)-S-(2-amino-2-carboxyethyl)-L-homocysteine, (*R*)-2-amino-3-sulfopropionic acid, D-2-amino-4-(ethylthio)butyric acid, 3-sulfino-L-alanine, Fmoc-Cys(Boc-methyl)-OH, seleno-L-cystine, *S*-(2-thiazolyl)-L-cysteine, *S*-(2-thienyl)-L-cysteine, *S*-(4-tolyl)-L-cysteine, *etc.,* but are not limited thereto.

As long as cysteine is produced according to the method of the present disclosure, cysteine can be converted to various cysteine derivatives, and the conversion to cysteine derivatives can be easily achieved by way of methods well known in the art.

Specifically, the method of producing cysteine derivatives may further include converting the cysteine produced in Step b) into a cysteine derivative. For example, cysteine may be synthesized into *N*-acetylcysteine (NAC) by a reaction with an acetylation agent, or it may be synthesized into *S*-carboxymethylcysteine (SCMC) by a reaction with a haloacetic acid under basic conditions, but the method is not limited thereto.

These cysteine derivatives are used mainly as pharmaceutical materials for antitussive agents, cough-relieving agents, and therapeutic agents for bronchitis, bronchial asthma, laryngopharyngitis, *etc.,* but are not limited thereto.

As used herein, the term "*O*-phosphoserine sulfhydrylase (OPSS)" refers to an enzyme that catalyzes the reaction by which OPS is converted into cysteine by imparting a thiol group (SH group) to OPS. The enzyme may have been first found in *Aeropyrum pernix*, *Mycobacterium tuberculosis, Mycobacterium smegmatis,* and *Trichomonas vaginalis* (Mino, K. and Ishikawa, K., FEBS Letters, 551:133-138, 2003; Burns, K. E. et al., J. Am. Chem. Soc., 127:11602-11603, 2005). Additionally, the OPSS may include not only wild-type OPSS proteins, but also variant proteins that include deletion, substitution, or addition in part of the sequence in the polynucleotide sequence encoding the OPSS, which show activity that is equal to or higher than the biological activity of the wild-type OPSS proteins, and may also include all the OPSS proteins disclosed in European Patent No. 2444481 and US Patent No. 9127324, and their protein variants.

The sulfide may be any sulfide provided not only in a solid form generally used in the art, but also in a liquid or gas form due to the difference in pH, pressure, and solubility, and thus can be converted to a thiol (SH) group in the form of sulfide (S²⁻) or thiosulfate (S₂O₃²⁻). Specifically, the sulfide may include Na₂S, NaSH, H₂S, (NH₄)₂S, and Na₂S₂O₃, which can provide a thiol group to OPS, but is not limited thereto. In the reaction, a single thiol group is provided to a single reactive OPS group to produce a single cysteine or a derivative thereof. In this reaction, a sulfide is specifically added in an amount of 0.1 to 3 molar equivalents, and specifically 1 to 2 molar equivalents based on the molar concentration of OPS, but is not limited thereto.

Further, the method of the present disclosure may further include a step of recovering the cysteine produced in the above reaction step. In particular, the desired cysteine may be recovered by separation and purification from the reaction solution using a suitable reaction known in the art.

In yet another aspect of the present disclosure, the present disclosure provides the use for the production of *O*-phosphoserine, cysteine, or cysteine derivatives of a recombinant microorganism for producing *O*-phosphoserine in which the activity of mdtH protein is enhanced compared to its endogenous activity.

In even another aspect of the present disclosure, the present disclosure provides the use for exporting *O*-phosphoserine of the mdtH protein from a microorganism.

The mdtH protein, endogenous activity, enhancement, *O*-phosphoserine, cysteine, cysteine derivatives, and microorganism are as described above.

### [Mode for Carrying Out the Invention]

Hereinafter, the present invention will be described in more detail by way of Examples. However, it is apparent to those skilled in the art to which the present disclosure belongs that these Examples are provided with for illustrative purposes only, and that the scope of the invention is not intended to be limited to or by these Examples.

### Example 1: Identification of mdtH membrane proteins

In order to identify *E. coli* membrane proteins involved in the export of OPS, screening was performed using a genomic DNA library of *Escherichia coli* W3110 (ATCC27325), an *E. coli* wild-type strain.

In order to set up the conditions under which the growth of *E. coli* was inhibited by OPS, a reference strain for producing OPS was prepared. The screening reference strain was an OPS-producing strain mutated to weaken the activity of endogenous phosphoserine phosphatase (serB) in W3110, and was named CA07-0012 (KCCM11212P, European Patent No. 2444481; U.S. Patent Application Publication No. 2012-0190081).

CA07-0012 was cultured in a medium containing OPS to establish optimal screening conditions showing growth inhibition. The W3110 genome library plasmid was transformed into CA07-0012 by electroporation (van der Rest *et al.* 1999), and colonies in which the growth inhibition was released in the medium condition supplemented with an excess of OPS were selected. Plasmids were obtained from the selected colonies, and nucleotide sequences were analyzed through sequencing techniques. From this, two types of *E. coli* membrane proteins involved in releasing the growth inhibition under the condition supplemented with an excess of OPS were identified.

As a result, the gene encoding the *E. coli* membrane protein (SEQ ID NO: 1) was identified as an mdtH transporter belonging to the major facilitator superfamily (MFS).

### Example 2: Preparation of mdtH overexpression vectors

When mdtH, which is involved in the release of the growth inhibition caused by OPS, was enhanced in each of the OPS-producing strains, overexpression vectors were prepared for each gene to confirm whether the OPS-exporting ability was improved. In addition, when yhhS, which is a phosphoserine exporter, was enhanced in the OPS-producing strains, it was confirmed that the OPS concentration was increased (WO 2016/024771 A1), and accordingly, this was used as a positive control. Further, the *E. coli* membrane protein yfaV MFS transporter belonging to the major facilitator superfamily (MFS) was also evaluated in the same manner as mdtH. A DNA fragment encoding mdtH was obtained through PCR using the W3110 genomic DNA as a template (SEQ ID NO: 2). The primer sequences used to prepare the overexpression vectors for each membrane protein gene are shown in Table 1 below.

**[Table 1]**

| SEQ ID NO: | Sequence Name | Primer | Gene | Vector |
|---|---|---|---|---|
| 11 | yhhS_ Ptrc_F | CGGGGATCCTCTAGACGCTTGCTGCAA CTCTCTCA | yhhS | pCL_Ptrc -yhhS |
| 12 | yhhS_ Ptrc_R | TACGGGTTCGGGcatGATATCTTTCCTG TGTGAAA | | |
| 13 | yhhS_F | CACAGGAAAGATATCatgCCCGAACCCG TAGCCGA | | |
| 14 | yhhS_R | GATTACGCCAAGCTTttaAGATGATGAG GCGGCCT | | |
| 15 | mdtH_ Ptrc_F | CGGGGATCCTCTAGACGCTTGCTGCAA CTCTCTCA | mdtH | pCL_Ptrc -mdtH |
| 16 | mdtH_ Ptrc_R | CGACACGCGGGAcatGATATCTTTCCTG TGTGAAA | | |
| 17 | mdtH_F | CACAGGAAAGATATCatgTCCCGCGTGT CGCAGGC | | |
| 18 | mdtH_R | GATTACGCCAAGCTTtcaGGCGTCGCGT TCAAGCA | | |
| 19 | yfaV_ Ptrc_F | CGGGGATCCTCTAGACGCTTGCTGCAA CTCTCTCA | yfaV | pCL_Ptrc -yfaV |
| 20 | yfaV_ Ptrc_R | CAAAGCGGTGCTcatGATATCTTTCCTGT GTGAAA | | |
| 21 | yfaV_F | CACAGGAAAGATATCatgAGCACCGCTT TGCTTGA | | |
| 22 | yfaV_R | GATTACGCCAAGCTTttaATGATGTGCCA CGTCGG | | |

In order to prepare pCL_Ptrc-yhhS, pCL_Ptrc-gfp (WO 2016/024771 A1) was used as a template, and PCR was performed using SEQ ID NO: 11 and SEQ ID NO: 12 to obtain Ptrc DNA fragments. The yhhS DNA fragments were obtained via PCR using SEQ ID NO: 13 and SEQ ID NO: 14 based on W3110 as a template. The amplified fragments were subjected to IST with the pCL1920 vector treated with restriction enzymes Xbal and HindIII to thereby obtain pCL_Ptrc-yhhS. IST (Gibson, D. G. et al., NATURE METHODS, Vol. 6 No. 5, MAY 2009, NEBuilder HiFi DNA Assembly Master Mix) refers to a method of cloning using the Gibson assembly method, and is continuously referred to as IST below.

For preparation of pCL_Ptrc-mdtH, pCL_Ptrc-gfp was also used as a template, and Ptrc DNA fragments were obtained using SEQ ID NOS: 15 and 16. The mdtH DNA fragments were obtained using SEQ ID NO: 17 and SEQ ID NO: 18 based on W3110 as a template, and pCL_Ptrc-mdtH was prepared through IST in the same manner as pCL_Ptrc-yhhS.

pCL_Ptrc-yfaV was also cloned in the same manner as in the preparation of the two plasmids, and the Ptrc DNA fragments were obtained using pCL_Ptrc-gfp as a template, and the yfaV DNA fragments were obtained using W3110 as a template. As primers, PCR was performed using SEQ ID NO: 19 and SEQ ID NO: 20 for Ptrc, and PCR was performed using SEQ ID NO: 21 and SEQ ID NO: 22 for yfaV.

### Example 3: Preparation of mdtH MFS transporter-enhanced strains and evaluation of OPS producing ability thereof

### 3-1. Preparation of mdtH MFS transporter-enhanced strains using CA07-0012 and evaluation of OPS producing ability thereof

Strains were prepared into which the three types of plasmids prepared in Example 2 were each introduced into the OPS-producing strain CA07-0012, and the OPS producing ability thereof was evaluated.

Each of the strains was plated out on a solid LB medium and then cultured in a 33°C incubator overnight. The strains cultured in the solid LB medium overnight were inoculated into a 25 mL titer medium shown in Table 2 above and then cultured in a 33°C incubator at a rate of 200 rpm for 48 hours. The OPS producing ability thereof is shown in Table 3.

**[Table 2]**

| Medium Composition | Amount |
|---|---|
| Glucose | 40 g |
| KH₂PO₄ (KP1) | 6 g |
| (NH₄)₂SO₄ | 17 g |
| MgSO₄·7H₂O | 1g |
| MnSO₄·4H₂O | 5 mg |
| FeSO₄·7H₂O | 10 mg |
| L-Glycine | 2.5 g/L |
| Yeast Extract | 3 g/L |
| CaCO₃ | 30 g/L |
| pH | 6.8 |

**[Table 3]**

| Strain | OD_{562 nm} | Glucose Consumption (g/L) | *O*-Phosphoserine (g/L) |
|---|---|---|---|
| CA07-0012/pCL1920 | 50.6 | 40 | 1.3 |
| CA07-0012/pCL_Ptrc-yhhS | 45.1 | 40 | 2.3 |
| CA07-0012/pCL_Ptrc-mdtH | 38.2 | 40 | 2.4 |
| CA07-0012/pCL_Ptrc-yfaV | 39.5 | 40 | 1.3 |

As can be seen in Table 3, among the cases in which the *E. coli* membrane protein gene was additionally introduced into the *E.* coli-derived CA07-0012 strain, the production of OPS was increased in the yhhS- and mdtH-enhanced strains, compared to CA07-0012. In particular, it was confirmed that the OPS concentration was increased by approximately 2-fold in the mdtH-enhanced strain of the present disclosure. In contrast, in the case of the yfaV-enhanced strain, which is a comparison group, the production of OPS was not increased.

Accordingly, the CA07-0012/pCL_Ptrc-mdtH was named CA07-0354. The CA07-0354 strain was deposited at the Korean Culture Center of Microorganisms (KCCM) under the Budapest Treaty on August 28, 2020, with Accession No. KCCM12781P.

### 3-2. Preparation of mdtH MFS transporter-enhanced strains using serA- and serC-enhanced strains, and evaluation of OPS producing ability thereof

CA07-0022 (KCCM1 1 103P, US patent No. 9689009), an OPS-producing strain with increased OPS producing ability via enhancement of the activity of serA (3-phosphoglycerate dehydrogenase) and serC (3-phosphoserine aminotransferase), which are OPS biosynthetic pathways, was used to determine the effect of the *E. coli* membrane protein gene, and overexpression vectors for the membrane protein gene were prepared in combination with serA and serC. The primer sequences used are as shown in Table 4 below.

**[Table 4]**

| SEQ ID NO: | Sequence Name | Primer | Gene | Vector |
|---|---|---|---|---|
| 23 | serA*,serC_ Ptrc_F | CGGGGATCCTCTAGAGGTACCCGCTT GCTGCAACT | serA*,serC | pCL_Ptrc-serA*C |
| 24 | serA*,serC_ Ptrc_R | CGATACCTTTGCCATGATATCTTTCCT GTGTGAAA | | |
| 25 | serA*,serC_F | CACAGGAAAGATATCATGGCAAAGGT | | |
| | | ATCGCTGGA | | |
| 26 | serA*,serC_R | GATTACGCCAAGCTTTTAACCGTGAC GGCGTTCGA | | |
| 27 | serA*,serC,yh hS_ Ptrc-yhhS_F | CACGGTTAAAAGCTTCGCTTGCTGCA ACTCTCTCA | serA*,serC, yhhS | pCL_Ptrc-serA*C_ Ptrc-yhhS |
| 28 | serA*,serC,yh hS_ Ptrc-yhhS_R | GATTACGCCAAGCTTttaAGATGATGAG GCGGCCT | | |
| 29 | serA*,serC,md tH_ Ptrc-mdtH_F | CACGGTTAAAAGCTTCGCTTGCTGCA ACTCTCTCA | serA*,serC, mdtH | pCL_Ptrc-serA*C_ Ptrc-mdtH |
| 30 | serA*,serC,md tH_ Ptrc-mdtH_R | GATTACGCCAAGCTTtcaGGCGTCGCG TTCAAGCA | | |
| 31 | serA*,serC,md tH_ Ptrc-yfaV_F | CACGGTTAAAAGCTTCGCTTGCTGCA ACTCTCTCA | serA*,serC, yfaV | pCL_Ptrc-serA*C_ Ptrc-yfaV |
| 32 | serA*,serC,md tH_Ptrc-yfaV_R | GATTACGCCAAGCTTttaATGATGTGCC ACGTCGG | | |

First, pCL_Ptrc-serA*C was prepared in order to prepare negative control plasmids into which serA and serC were introduced. The Ptrc DNA fragments were obtained using SEQ ID NO: 23 and SEQ ID NO: 24 based on pCL_Ptrc-gfp as a template. PCR was performed using SEQ ID NO: 25 and SEQ ID NO: 26 based on pC_Prmf-serA*C (WO 2016/024771 A1) as a template to obtain serA*C DNA fragments. The amplified fragments were subjected to IST with the pCL1920 vector treated with restriction enzymes Xbal and HindIII to obtain pCL_Ptrc-serA*C.

For preparation of pCL_Ptrc-serA*C_Ptrc-yhhS, PCR was performed using SEQ ID NO: 27 and SEQ ID NO: 28 based on pCL_Ptrc-yhhS, which was prepared above, as a template. From this, Ptrc-yhhS DNA fragments were obtained. The amplified fragments were subjected to IST with the pCL_Ptrc-serA*C vector treated with HindIII to obtain pCL_Ptrc-serA*C_Ptrc-yhhS.

pCL_Ptrc-serA*C_Ptrc-mdtH and pCL_Ptrc-serA*C_Ptrc-yfaV were also prepared in the same manner as pCL_Ptrc-serA*C_Ptrc-yhhS. The Ptrc-mdtH DNA fragments were obtained based on pCL_Ptrc-mdtH as a template using SEQ ID NO: 29 and SEQ ID NO: 30, and Ptrc-yfaV DNA fragments were obtained based on pCL_Ptrc-yfaV as a template using SEQ ID NO: 31 and SEQ ID NO: 32.

The OPS producing ability was confirmed by introducing the prepared plasmids into CA07-0022, an OPS-producing strain, and the results are shown in Table 5 below.

**[Table 5]**

| Strain | OD_{562 nm} | Glucose Consumption (g/L) | O-Phosphoserine (g/L) |
|---|---|---|---|
| CA07-0022/pCL_Ptrc-serA*C(se rA*(G336V)-(RBS)serC | 45.0 | 40 | 3.5 |
| CA07-0022/pCL_Ptrc-serA*C_Pt rc-yhhS | 35.1 | 40 | 7.6 |
| CA07-0022/pCL_Ptrc-serA*C_Pt rc-mdtH | 30.0 | 40 | 7.8 |
| CA07-0022/pCL_Ptrc-serA*C_Pt rc-yfaV | 25.9 | 40 | 3.2 |

As can be seen in Table 5 above, among the strains in which the *E. coli* membrane protein gene was additionally introduced into CA07-0022/pCL_Ptrc-serA*C, which has improved OPS producing ability compared to the CA07-0012 strain derived from *E. coli,* it was confirmed once again that the production of OPS was increased in the yhhS-enhanced strain, which was the positive control, compared to the control. In particular, in the case of the mdtH-enhanced strain according to the present disclosure, the OPS concentration was increased, similar to the results shown in Table 3. In contrast, in the case of the yfaV-enhanced group, which is the comparison group, the OPS production was decreased compared to the control group.

### 3-3. Preparation of mdtH transporter-enhanced strain according to chromosomal promoter strength and evaluation of OPS producing ability thereof

In order to confirm whether the exporting ability was improved when the promoter of mdtH was replaced with a stronger promoter on the chromosome, strains were prepared in which the autologous promoter was substituted with the trc promoter, and their OPS producing ability was evaluated. The method of introducing the trc promoter into the chromosome of *E. coli* was carried out by way of the following commonly used method. For chromosomal insertion, the pSKH130 vector having a PI protein (pir gene)-dependent R6K replicon and into which the sacB (Levansucrase) gene is introduced was used. In addition, the vector contains a kanamycin resistance gene, which was used as a selection marker for the preparation of strains. After obtaining the desired strain using R6K and kanamycin at the first crossover using the vector, the antibiotics were removed from the medium containing sucrose to prepare strains. In order to replace the promoter of mdtH with trc in CA07-0022, the pSKH130_Ptrc-mdtH vector was prepared, and the primer sequences used are shown in Table 6 below.

**[Table 6]**

| SEQ ID NO: | Sequence Name | Primer | Gene | Vector |
|---|---|---|---|---|
| 33 | mdtHUP_F | CAGGAATTCGATATCTAATCTCTTTTT CGTCCGGG | mdtH | pSKH130_ mdtHUP_Pt rc-mdtH |
| 34 | mdtHUP_R | GAGTTGCAGCAAGCGTTCCCCTCCC GGGAAATAAA | | |
| 35 | Ptrc-mdtH_ F | TTCCCGGGAGGGGAACGCTTGCTGC AACTCTCTCA | | |
| 36 | Ptrc-mdtH_ R | GACTAGCGTGATATCCAGACCAGGC GAAAGTCGTA | | |
| 37 | Ptrc-mdtH_ conf_F | CACCGCTGCGTTTATTGT | | |
| 38 | Ptrc-mdtH_ conf_R | AAACGCTTGTCACGCATCA | | |

For preparation of pSKH130_Ptrc-mdtH, the mdtHUP DNA fragments were obtained by performing PCR based on W31 10 using SEQ ID NO: 33 and SEQ ID NO: 34. In addition, Ptrc-mdtH DNA fragments were obtained by performing PCR based on the pCL_Ptrc-mdtH prepared above using SEQ ID NO: 35 and SEQ ID NO: 36. The amplified fragments were subjected to IST with the pSKH130 vector treated with restriction enzyme EcoRV to obtain pSKH_mdtHUP_Ptrc-mdtH. The thus-obtained plasmids were transformed into the CA7-0022 strain via electroporation. After selecting the strain introduced into the chromosomes in the LB solid medium supplemented with kanamycin via recombination (crossover), the plasmid region was excised from the chromosome through secondary recombination (replacement) in the medium containing sucrose. The strain in which the secondary recombination was completed was obtained using the primers of SEQ ID NO: 37 and SEQ ID NO: 38 (CA07-0022::Ptrc-mdtH).

In order to determine the effect of the prepared OPS-producing strains, the OPS producing ability is shown in Table 7 below.

**[Table 7]**

| Strain | OD_{562 nm} | Glucose Consumption (g/L) | *O*-Phosphoserine (g/L) |
|---|---|---|---|
| CA07-0022/pCL_Ptrc-serA*C | 42.4 | 40 | 3.5 |
| CA07-0022::Ptrc-mdtH/pCL_Ptrc-serA*C | 38.2 | 40 | 4.0 |

As a result, as can be seen in Table 7, when the expression of the *E. coli* membrane protein was increased by enhancing the promoter, it was confirmed that the OPS concentration was increased compared to the control group.

### Example 4: Comparison of 3-phosphoglycerate export

OPS is a substance containing phosphate and has a chemical structure similar to 3-phosphoglycerate (hereinafter referred to as 3PG). Accordingly, it was assumed that the OPS exporter could release 3PG, and the 3PG producing ability was measured in the strains enhanced with the OPS exporter. 3PG was measured using a high-performance liquid chromatography (HPLC) instrument, and the 3PG producing ability is shown in Table 8.

**[Table 8]**

| Strain | OD_{562 nm} | OPS (g/L) | 3PG (g/L) |
|---|---|---|---|
| CA07-0022/pCL_Ptrc-serA*C | 45.0 | 3.5 | 0.2 |
| CA07-0022/pCL_Ptrc-serA*C_Ptrc-yhh S | 35.1 | 7.6 | 2.6 |
| CA07-0022/pCL_Ptrc-serA*C_Ptrc-mdt H | 30.0 | 7.8 | 0.3 |
| CA07-0022/pCL_Ptrc-serA*C_Ptrc-yfa V | 25.9 | 3.2 | 0.3 |

As can be seen in Table 8, 3PG was accumulated in the yhhS-enhanced strain, but 3PG was not increased in the mdtH membrane protein-enhanced strain. That is, it was confirmed that the strain into which mdtH was introduced had increased OPS exporting ability, and that the exporting ability specific for OPS was also increased.

Those of ordinary skill in the art will recognize that the present disclosure may be embodied in other specific forms without departing from its spirit or essential characteristics. The described embodiments are to be considered in all respects only as illustrative and not restrictive. The scope of the present disclosure is therefore indicated by the appended claims rather than by the foregoing description. All changes which come within the meaning and range of equivalency of the claims are to be embraced within the scope of the present disclosure.

### [Deposition No.]

Depository Institution: Korean Culture Center of Microorganisms (International Depositary Authority)
Accession No.: KCCM12781P
Deposition Date: 20200828

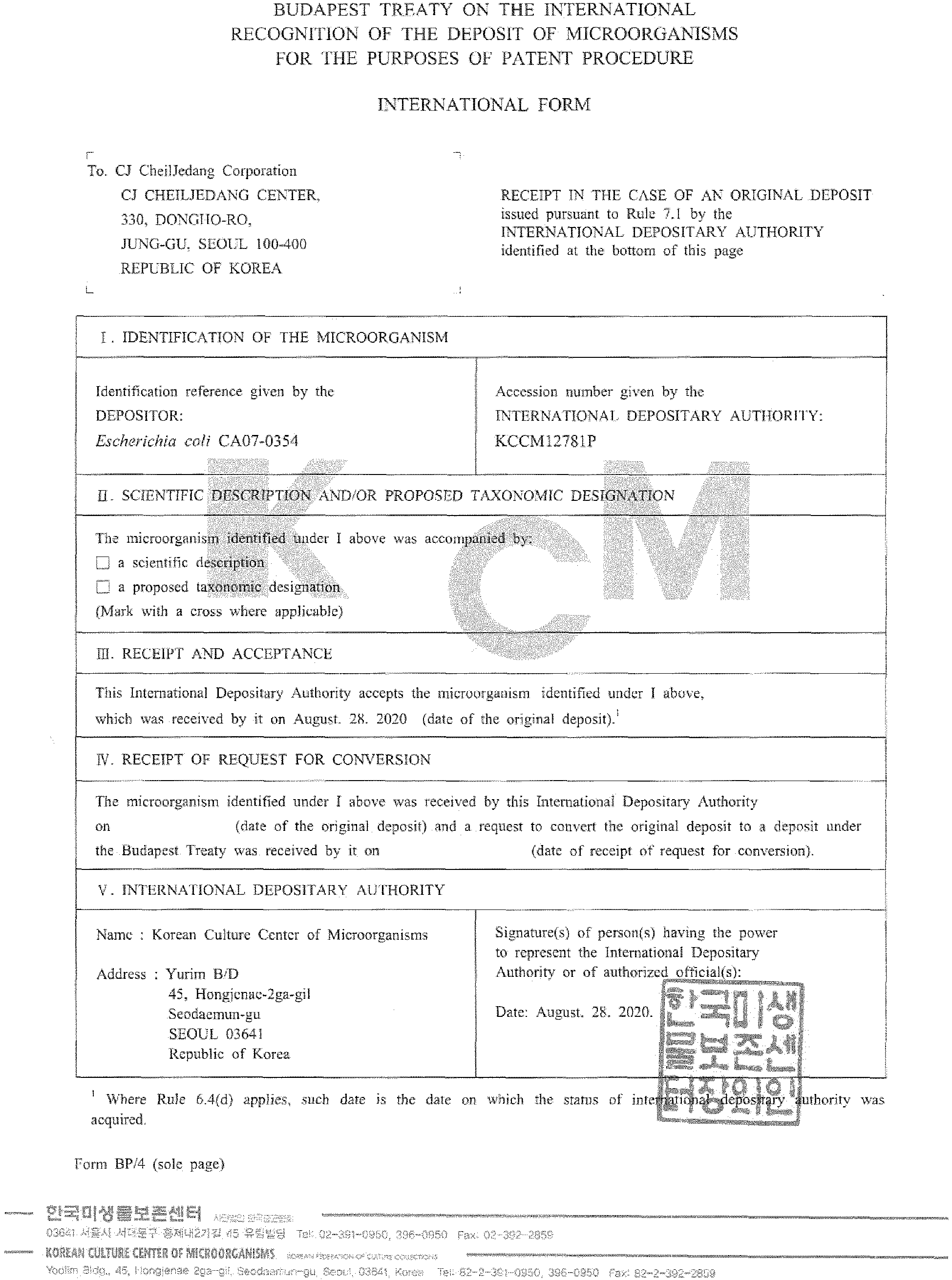
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Escherichia coli CA07-0354 | Accession number given by the
INTERNATIONAL DEPOSITARY AUTHORITY:
KCCM12781P

II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)

III. RECEIPT AND ACCEPTANCE
This International Depositary Authority accepts the microorganism identified under I above,
which was received by it on August. 28. 2020 (date of the original deposit).¹

IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: August. 28. 2020.

¹ Where Rule 6.4(d) applies, such date is the date on which the status of international depositary authority was
acquired.

Form BP/4 (sole page)

한국미생물보존센터 [?]
03641 서울시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yoolim Bldg., 45, Hongjenae 2ga-gil, Seodaemun-gu, Seoul, 03641, Korea Tel: 82-2-391-0950, 396-0950 Fax: 82-2-392-2859

## Claims

1. A recombinant microorganism for producing *O*-phosphoserine in which the activity of mdtH protein is enhanced compared to its endogenous activity.

2. The microorganism of claim 1, in which the *O*-phosphoserine exporting activity is enhanced compared to its endogenous activity.

3. The microorganism of claim 1, wherein the mdtH protein comprises an amino acid sequence of SEQ ID NO: 1, or an amino acid sequence having at least 95% identity with SEQ ID NO: 1, while having the *O*-phosphoserine exporting activity.

4. The microorganism of claim 1, in which the activity of phosphoserine phosphatase (SerB) is further weakened compared to its endogenous activity.

5. The microorganism of claim 1, in which the activity of any one of phosphoglycerate dehydrogenase (SerA), phosphoserine aminotransferase (SerC), or a combination thereof is further enhanced compared to its endogenous activity.

6. The microorganism of claim 1, wherein the recombinant microorganism belongs to the genus *Escherichia.*

7. A method for producing *O*-phosphoserine, comprising culturing a recombinant microorganism for producing *O*-phosphoserine in which the activity of mdtH protein is enhanced compared to its endogenous activity, in a medium.

8. The method of claim 7, wherein the method further comprises recovering O-phosphoserine in the cultured medium or the microorganism.

9. A method for producing cysteine or derivatives thereof, comprising:
a) producing *O*-phosphoserine or a medium containing *O*-phosphoserine by culturing a recombinant microorganism for producing *O*-phosphoserine in which the activity of mdtH protein is enhanced compared to its endogenous activity, in a medium; and
b) reacting the *O*-phosphoserine or the medium containing the same produced in Step a) with a sulfide, in the presence of *O*-phosphoserine sulfhydrylase (OPSS) or a microorganism containing the same.

10. The method of claim 9, wherein the method for producing cysteine derivatives further comprises converting cysteine produced in Step b) into cysteine derivatives.

11. The method of claim 9, wherein the sulfide is at least one selected from the group consisting of Na₂S, NaSH, (NH₄)₂S, H₂S, and Na₂S₂O₅.

12. Use of a recombinant microorganism for the production of O-phosphoserine for producing O-phosphoserine, cysteine, or a derivative of cysteine, in which the activity of a mdtH protein is enhanced compared to its endogenous activity.

13. Use of a mdtH protein for exporting O-phosphoserine from a microorganism.
